# EUROPÄISCHE PATENTSCHRIFT

(11) **EP 2 042 203 B1**
(45) Veröffentlichungstag und Bekanntmachung des Hinweises auf die Patenterteilung: **15.01.2014**
(21) Anmeldenummer: 08016818.0
(22) Anmeldetag: 25.09.2008
(51) Int. Cl.: A61L 31/14, A61L 31/16, A61L 31/10

(54) **Verstärkte Gefässprothese mit antimikrobieller Langzeitwirkung**
Reinforced stent with antimicrobial long-term effect
Prothèse vasculaire renforcée ayant un effet antimicrobien prolongé

(30) Priorität: 26.09.2007 DE 102007047246
(43) Veröffentlichungstag der Anmeldung: 01.04.2009
(73) Patentinhaber: Aesculap AG, 78532 Tuttlingen (DE)
(72) Erfinder: Goldmann, Helmut, Dr., 78532 Tuttlingen (DE); Langanke, Dennis, Dr., 78532 Tuttlingen (DE)
(74) Vertreter: Patentanwälte Ruff, Wilhelm, Beier, Dauster & Partner

(56) Entgegenhaltungen:
- WO-A2-99/09911
- US-A1- 2005 283 224

## Beschreibung

Die Erfindung betrifft eine an ihrer Außenoberfläche verstärkte Gefäßprothese mit einem antimikrobiellen Langzeitschutz.

Die Infektion bei der Implantation von Gefäßprothesen und anderen Implantaten stellt eine Komplikation dar, die sowohl von Ärzten als auch von Patienten gefürchtet ist. Die Häufigkeit einer Implantatinfektion beträgt etwa 0,5 bis 5 %. Risikofaktoren stellen bei künstlichen Gefäßimplantaten zum Beispiel die subkutane Positionierung von Gefäßprothesen oder deren Positionierung in der Leistengegend dar. Bei den auftretenden Infektionen kann es sich um sogenannte Früh- oder Spätinfektionen handeln. Frühinfektionen treten in der Regel während eines Zeitraums von bis zu 4 Monaten nach der Implantation auf. Dagegen treten Spätinfektionen in der Regel erst in größeren Zeitabschnitten nach der Implantation auf. Beispielsweise existieren klinische Berichte, denen zufolge Infektionen der Aorta noch nach einem Zeitraum von 25 bis 70 Monaten aufgetreten sind. In aorto-femoraler Position beträgt die mittlere Zeit bis zum Ausbruch der Infektion sogar 41 Monate.

Zu den relevanten Erregern zählen vor allem Staphylococcus aureus, Staphylococcus epidermidis, Methicillin resistente Staphylococcus aureus (MRSA) und Escherichia coli. Die Infizierung erfolgt gewöhnlich durch intraoperative Kontamination. Sie kann aber auch postoperativ erfolgen, insbesondere bei einer nicht völlig ausgeheilten Infektion des Patienten. Implantate mit einer antimikrobiellen Langzeitwirkung gewinnen daher zunehmend an Bedeutung. Ein diesbezüglich geeignetes Implantat ist beispielsweise aus der DE 103 23 676 A1 bekannt.

Neben einem antimikrobiellen Langzeitschutz sollten die Implantate auch über eine ausreichende mechanische Belastbarkeit verfügen. Dies gilt vor allem für Gefäßprothesen, die Gelenk überschreitend oder als extraanatomischer Bypass implantiert werden. In diesem Fall ist vor allem eine ausreichende Knick- und Kompressionsstabilität erwünscht. Knickstabile Gefäßprothesen sind an sich bekannt und werden beispielsweise in der WO 96/4001 A1, WO 97/33533 A1, EP 0 699 424 A2 und der US 2005/0096737 A1 beschrieben.

Aus der US 2005/0283224 A1 ist eine antimikrobiell additivierte Gefäßprothese mit einer Texilschicht als Verstärkungsmittel bekannt. Die WO 99/09911 A2 betrifft einen Stent mit einem fadenförmigen Verstärkungsmittel.

Die vorliegende Erfindung stellt sich nun die Aufgabe, eine Gefäßprothese mit einem antimikrobiellen Schutz bereitzustellen, vor allem auch gegenüber möglichen Spätinfektionen, die gleichzeitig über ausreichend knickstabile Eigenschaften verfügt.

Diese Aufgabe wird erfindungsgemäß durch eine Gefäßprothese mit einer Prothesenwand gelöst, deren Außenoberfläche zumindest ein Verstärkungsmittel aufweist, wobei die Oberfläche der Prothesenwand und die Oberfläche des zumindest einen Verstärkungsmittels zumindest zum Teil eine antimikrobiell wirksame Schicht aufweisen. Gegebenenfalls kann die Schicht mehrere antimikrobiell wirksame Schichten umfassen.

Bei der Oberfläche der Prothesenwand, die zumindest zum Teil eine antimikrobiell wirksame Schicht aufweist, handelt es sich erfindungsgemäß vorzugsweise um deren Außenoberfläche, d.h. um die zur Außenseite der Prothese gerichtete Oberfläche der Prothesenwand. Alternativ oder in Kombination kann es sich bei der Oberfläche des Verstärkungsmittels insbesondere um dessen Außenoberfläche, d.h. die zur Außenseite der Prothese gerichtete Oberfläche, handeln.

In einer besonders bevorzugten Ausführungsform ist das zumindest eine Verstärkungsmittel ein wendelförmig in Längsrichtung der Gefäßprothese verlaufendes Verstärkungsmittel.

Weiterhin kann es erfindungsgemäß vorgesehen sein, dass das zumindest eine Verstärkungsmittel ringförmig auf der Außenoberfläche der Prothesenwand ausgebildet ist. Insbesondere kann die Gefäßprothese an ihrer Außenoberfläche mehrere ringförmig ausgebildete Verstärkungsmittel aufweisen. Die ringförmigen Verstärkungsmittel können in regelmäßigen oder unregelmäßigen Abständen auf der Außenoberfläche der Prothesenwand angeordnet sein. Bevorzugt weisen die ringförmigen Verstärkungsmittel untereinander einen konstanten Abstand auf. Die Anzahl der ringförmig ausgebildeten Verstärkungsmittel kann variieren und ist insbesondere abhängig von der Prothesenlänge. Der Innendurchmesser der ringförmigen Verstärkungsmittel ist ebenfalls vorzugsweise variabel und insbesondere davon abhängig, welchen Außendurchmesser die Gefäßprothese aufweist. In dieser Ausführungsform liegen die ringförmigen Verstärkungsmittel bevorzugt in Form von Ringen vor, welche jeweils die Gefäßprothese an deren Außenoberfläche in bestimmten Abständen umgeben.

Überraschenderweise stellte sich heraus, dass eine verstärkte Gefäßprothese auch im Bereich ihres Verstärkungsmittels dauerhaft mit einer antimikrobiellen Ausrüstung versehen werden kann, ohne die mechanische Eigenschaften, die die Gefäßprothese aufgrund ihrer Armierung mit dem Verstärkungsmittel erhält, zu beeinträchtigen. Durch die Erfindung wird daher eine Gefäßprothese bereitgestellt, bei der sowohl die Prothesenwand als auch das zumindest eine Verstärkungsmittel einen Infektionsschutz bieten. Dadurch kann die Gefäßprothese in einer besonders vorteilhaften Ausführungsform an ihrer kompletten Außenoberfläche antimikrobiell ausgerüstet sein und auf diese Weise die Etablierung von pathogenen Erregern in der Prothesenumgebung auch langfristig verhindern. Das zumindest eine Verstärkungsmittel schützt die Gefäßprothese dabei vor Druckbelastungen und einem Abknicken der Prothese.

In einer bevorzugten Ausführungsform ist die Oberfläche des zumindest einen Verstärkungsmittels vollständig mit der antimikrobiell wirksamen Schicht beschichtet. In einer weiteren Ausführungsform ist die Außenoberfläche der Prothese, insbesondere der Prothesenwand, vollständig mit der Schicht beschichtet. Bevorzugt befindet sich an den Stellen, insbesondere wendelförmig verlaufenden Stellen, zwischen der Prothesenwand und dem zumindest einen Verstärkungsmittel, insbesondere wendelförmig verlaufenden Verstärkungsmittel, zumindest eine antimikrobiell wirksame Schicht. Dies ist besonders bei der Durchführung von Anastomosen von Vorteil, wo die Prothese üblicherweise mit einem natürlichen Gefäßabschnitt vernäht wird. Hierzu wird in der Regel ein Stück des zumindest einen Verstärkungsmittels von der übrigen Prothese abgetrennt, wodurch die darunterliegenden Bereiche der Außenoberfläche der Prothesenwand freigelegt werden. Durch die Beschichtung dieser Außenoberflächenbereiche ist in diesem Fall ein vollständiger Infektionsschutz gewährleistet. Bei Bedarf kann die Gefäßprothese auch an der Innenoberfläche der Prothesenwand antimikrobiell beschichtet sein. Diesbezüglich wird auf die DE 103 23 676 A1 verwiesen.

In einer möglichen Ausführungsform können die im vorherigen Abschnitt genannten Stellen zwischen der Prothesenwand und dem zumindest einen Verstärkungsmittel frei von einer antimikrobiell wirksamen Schicht sein. In dieser Ausführungsform ist nur die Außenoberfläche, d.h. die zur Außenseite der Gefäßprothese gerichtete Oberfläche, des zumindest einen Verstärkungsmittels antimikrobiell beschichtet.

Erfindungsgemäß kann die antimikrobiell wirksame Schicht verschiedene Schichtdicken an der Außenoberfläche der Gefäßprothese aufweisen. Die antimikrobiell wirksame Schicht kann insbesondere unterschiedliche Schichtstärken auf der Außenoberfläche der Prothesenwand und/oder auf der Oberfläche des zumindest einen Verstärkungsmittels aufweisen. So kann die Schichtdicke der antimikrobiell wirksamen Schicht auf der Prothesenwand größer als auf dem zumindest einen Verstärkungsmittel sein oder umgekehrt. Bevorzugt weist die antimikrobiell wirksame Schicht gleiche Schichtstärken auf der Außenoberfläche der Prothesenwand und auf der Oberfläche des zumindest einen Verstärkungsmittels auf.

In einer weitergehenden Ausführungsform weist die antimikrobiell wirksame Schicht eine Schichtdicke bis zu 400 nm auf. Bevorzugt weist die antimikrobiell wirksame Schicht eine Schichtdicke zwischen 40 nm und 200 nm, insbesondere 80 nm bis 160 nm, auf.

Das zumindest eine Verstärkungsmittel ist vorzugsweise dauerhaft auf der Außenoberfläche der Prothesenwand befestigt. Bevorzugt ist das zumindest eine Verstärkungsmittel auf die Prothesenwandaußenoberfläche aufgeklebt, beispielsweise mit Hilfe eines hierfür geeigneten Schmelzklebers.

In einer bevorzugten Ausführungsform ist das zumindest eine Verstärkungsmittel von filamentartiger Struktur. Erfindungsgemäß kann es sich bei dem zumindest einen Verstärkungsmittel um ein Mono- oder Multifilament handeln. Bevorzugter Weise ist das zumindest eine Verstärkungsmittel ein Monofilament. Erfindungsgemäß kann es weiterhin vorgesehen sein, dass das zumindest eine Verstärkungsmittel als Faden, Garn oder Draht vorliegt. Beispielsweise kann es sich bei dem Faden um einen Schmelzfaden handeln. Der Faden, insbesondere Schmelzfaden, ist weiterhin mit einem Schmelzkleber ummantelt. Mit anderen Worten handelt es sich bei dem zumindest einen Verstärkungsmittel um einen schmelzkleberummantelten Faden. Der Schmelzkleber kann insbesondere auf einem Copolymer mit Polybutylen basieren. Eine bandförmige Ausgestaltung des zumindest einen Verstärkungsmittels ist erfindungsgemäß ebenso möglich.

Das zumindest eine Verstärkungsmittel besteht vorzugsweise aus einem Kunststoff. Der Kunststoff kann resorbierbar oder vorzugsweise nicht resorbierbar sein. Bei dem Kunststoff kann es sich um einen Polyester oder um ein Polyolefin, vorzugsweise um Polypropylen, handeln. Besonders bevorzugt handelt es sich bei dem zumindest einen Verstärkungsmittel um einen Polypropylenfaden.

Als Material zur Herstellung der Prothesenwand kommen die üblicherweise bei Gefäßprothesen verwendeten Polymere in Frage, insbesondere Polyester, Polytetrafluorethylen (PTFE), Polyurethan sowie in besonderen Fällen auch Polyamide. Die Verwendung von Polyester, insbesondere Polyethylenterephthalat, und/oder Polytetrafluorethylen, insbesondere expandiertes Polytetrafluorethylen (ePTFE), ist besonders bevorzugt.

Die Prothesenwand weist vorzugsweise eine Wandstärke zwischen 0,1 und 1 mm, insbesondere zwischen 0,4 und 0,6 mm, auf. Die Prothesenwand und bevorzugt auch die antimikrobiell wirksame Schicht sind in einer weiteren Ausführungsform porös ausgebildet.

Handelt es sich bei der erfindungsgemäßen Gefäßprothese um eine gewirkte Gefäßprothese, kann diese beispielsweise durch Wirken in Einfach-Trikobindung ausgebildet sein. Bei der Einfach-Trikotbindung, auch Single-Trikotbindung genannt, wird mit nur einer Legeschiene gewirkt. Des weiteren kann die Gefäßprothese auch durch Wirken in Doppel-Trikotbindung ausgebildet sein. In der Regel wird hier mit zwei Legeschienen gewirkt. In einer weitergehenden Ausführungsform kann die Gefäßprothese durch Wirken in einer Kombination von Bindungstechniken ausgebildet sein. Als Beispiele solcher kombinierter Wirktechniken sind Bindungen Trikot-Atlas, Trikot-Franse, Franse/Schuss und Kombinationen mit Fileteinzug zu nennen.

Die Gefäßprothese kann grundsätzlich aus monofilen Fäden oder multifilen Fäden, insbesondere Multifilamentgarnen, gebildet sein. In der Regel weisen die Fäden dabei einen runden Querschnitt auf. Die Gefäßprothese kann eine Filamentanzahl zwischen 10 und 70, insbesondere 35 und 45, aufweisen. Des Weiteren kann die Gefäßprothese einen Garntiter zwischen 30 und 200 dtex, vorzugsweise 40 und 60 dtex, aufweisen.

In einer weiteren Ausführungsform weist die Prothese eine radiale Reißkraft ≥ 5 N/mm,insbesondere 10 und 25 N/mm, auf.

Bevorzugt handelt es sich bei der erfindungsgemäßen Gefäßprothese um eine textile Gefäßprothese. Grundsätzlich kann die Gefäßprothese als Gewirk, Gestrick, Geflecht, Gewebe oder Vlies vorliegen. Besonders bevorzugt ist die erfindungsgemäße Gefäßprothese eine gewirkte Gefäßprothese. Erfindungsgemäß kann es weiterhin vorgesehen sein, dass die Gefäßprothese eine Doppelveloursprothese ist.

In einer weiteren Ausführungsform ist die Gefäßprothese unplissiert.

Die antimikrobiell wirksame Schicht weist in aller Regel eine antimikrobiell wirksame Substanz auf. Erfindungsgemäß können auch Kombinationen von antimikrobiell wirksamen Substanzen in Frage kommen.

Grundsätzlich kann die antimikrobiell wirksame Schicht auf der Prothesenwandoberfläche andere antimikrobielle Substanzen als auf der Oberfläche des zumindest einen Verstärkungsmittels aufweisen oder umgekehrt. Bevorzugt weist die antimikrobiell wirksame Schicht auf der Prothesenwandoberfläche und der Oberfläche des zumindest einen Verstärkungsmittels die gleiche antimikrobielle Substanz bzw. die gleichen antimikrobiellen Substanzen auf. Besonders bevorzugt weist die antimikrobiell wirksame Schicht einen Anteil einer antimikrobiell wirksamen Substanz zwischen 0,05 und 1,0 Gew.%, insbesondere 0,10 und 0,40 Gew.%, bezogen auf das Gesamtgewicht der Gefäßprothese. In der Regel trägt zu dem Gesamtgewicht der Gefäßprothese auch eine Imprägnierungsbeschichtung bei, auf die im Folgenden noch näher eingegangen wird.

Als antimikrobiell wirksame Substanzen kommen besonders antimikrobiell wirksame Metalle und/oder deren Salze in Betracht. Als antimikrobiell wirksame Metalle kommen vor allem Platin, Iridium, Gold, Silber und/oder Kupfer in Frage. Silber ist bevorzugt. Bei den Metallsalzen kann es sich insbesondere um Metalloxide handeln. Die in Frage kommenden Salze sind vorzugsweise schwerlöslich. Besonders bevorzugt handelt es sich bei dem Salz um ein schwerlösliches Silbersalz. Bevorzugt handelt es sich bei der antimikrobiell wirksamen Schicht um eine metallische Schicht, insbesondere um eine Schicht aus metallischem Silber. Eine Metallschicht, insbesondere eine Silberschicht, kann mittels an sich bekannter Bedampfungsverfahren, zum Beispiel durch das sogenannte IBAD-Verfahren (ion-beam-assisted deposition-Verfahren) erzeugt werden. Besonders bevorzugt ist es, wenn die Metallatome der Metallschicht in die Außenoberflächen der Prothesenwand und/oder des zumindest einen Verstärkungsmittels eingeprägt sind. Dies kann insbesondere durch Beschuss dieser Außenoberflächen beispielsweise mit Argonionen während der Bedampfung bewerkstelligt werden.

Die antimikrobiell wirksame Schicht der Gefäßprothese ist vorzugsweise eine geschlossene Schicht. Dies bedeutet jedoch nicht, dass im Falle einer porösen Gefäßprothesenstruktur deren Poren durch die Schicht verschlossen werden. Vielmehr kann sich die antimikrobiell wirksame Schicht der Oberflächenstruktur der Gefäßprothese anpassen, so dass die Poren ihre ursprüngliche Form und Größe behalten.

Zwischen der Außenoberfläche der Prothesenwand und der antimikrobiell wirksamen Schicht kann erfindungsgemäß eine Grundierungsschicht vorliegen. Die Grundierungsschicht kann insbesondere aus Titan, Niob und/oder Palladium bestehen.

In einer bevorzugten Ausführungsform ist die Gefäßprothese imprägniert. Bei der Imprägnierung handelt es sich vorzugsweise um eine abdichtende Imprägnierung bzw. Beschichtung. Die Imprägnierung liegt zweckmäßigerweise in Form einer Imprägnierungsschicht vor. Als Imprägnierungsmaterialien kommen sinnvollerweise bioverträgliche Materialien in Betracht. Beispielsweise kann die Gefäßprothese mit biologischen Materialien imprägniert sein, insbesondere mit Collagen, Gelatine und/oder Albumin. Die biologischen Materialien sind bevorzugt xenogenen Ursprungs, insbesondere bovinen, porcinen und/oder equinen Ursprungs. Bevorzugt sind die Imprägnierungsmaterialien vernetzt. Hierfür eignen sich insbesondere di-, tri-, tetra- oder allgemein oligofunktionelle Verbindungen, beispielsweise Diisocyanate oder Glutaraldehyd. Weitere geeignete Imprägnierungsmaterialien stellen in-vivo abbaubare oder resorbierbare synthetische Polymere, insbesondere Copolymere, dar. Hier kommen neben mindestens teilweise wasserlöslichen Polymeren, wie Polyvinylalkohol und/oder Carboxymethylcellulose, vorwiegend Polymere und Copolymere auf der Basis von Hydroxycarbonsäuren in Frage. Bevorzugt ist die Gefäßprothese mit Polymeren bzw. Copolymeren auf der Basis von Glykolid, Lactid, ε-Caprolacton, Trimethylencarbonat und/oder p-Dioxanon imprägniert. Durch geeignete Wahl der Polymere kann die gewünschte Resorptionsdauer eingestellt werden. Diese kann insbesondere 4 Monate, vorzugsweise 40 Tage, betragen. Eine solche Zeit ist besonders günstig, da die imprägnierende Wirkung je nach Art der Gefäßprothese nach dieser Zeit aufgrund des eingewachsenen Bindegewebes nicht mehr erforderlich ist. Bevorzugt weist die Gefäßprothese eine abdichtende Imprägnierung auf, welche die antimikrobiell wirksame Schicht mit einschließt.

Die vorliegende Erfindung betrifft weiterhin ein Verfahren zur Herstellung der Gefäßprothese, umfassend die Schritte:
a) Aufbringen des zumindest einen Verstärkungsmittels, insbesondere eines länglichen Verstärkungsmittels, auf die Außenoberfläche einer Gefäßprothese unter Ausbildung einer an ihrer Außenseite verstärkten, insbesondere wendelförmig verstärkten, Gefäßprothese,
b) Beschichten der Gefäßprothese mit einer antimikrobiell wirksamen Substanz vor und/oder nach dem Aufbringen des zumindest einen Verstärkungsmittels, insbesondere des länglichen Verstärkungsmittels.

In einer möglichen Ausführungsform kann das zumindest eine Verstärkungsmittel unabhängig von der Gefäßprothese, d.h. vor dem Aufbringen auf die Außenoberfläche der Gefäßprothese, mit einer antimikrobiell wirksamen Substanz beschichtet werden.

Das Aufbringen des zumindest einen Verstärkungsmittels auf die Außenoberfläche der Gefäßprothese erfolgt vorzugsweise unter Spiralisierung der Gefäßprothese.
In einer weiteren möglichen Ausführungsform kann zur Aufbringung des zumindest einen Verstärkungsmittels auf die Außenoberfläche der Gefäßprothese ein Polymerfaden ausgesponnen und zweckmäßigerweise in einem noch feuchten Zustand auf die Gefäßprothese aufgewickelt werden.

In einer bevorzugten Ausführungsform umfasst das Herstellungsverfahren die Schritte:
a) Beschichten einer Gefäßprothese mit einer antimikrobiell wirksamen Substanz,
b) Aufbringen des zumindest einen Verstärkungsmittels auf die Außenoberfläche der beschichteten Gefäßprothese unter Ausbildung einer an ihrer Außenseite verstärkten beschichteten Gefäßprothese, wobei das zumindest eine Verstärkungsmittel vor dem Aufbringen bereits mit der antimikrobiell wirksamen Substanz beschichtet ist und/oder die beschichtete Gefäßprothese nach dem Aufbringen des zumindest einen Verstärkungsmittels nochmals beschichtet wird.

In einer bevorzugten Ausführungsform wird das zumindest eine Verstärkungsmittel mittels eines Klebers, vorzugsweise eines Schmelzklebers, auf die Außenoberfläche der Gefäßprothese aufgebracht. Das zumindest eine Verstärkungsmittel kann auch selbst von einem Schmelzkleber gebildet werden.

In einer weiteren Ausführungsform wird zur Beschichtung der Gefäßprothese mit der antimikrobiell wirksamen Substanz eine Metallbedampfung, insbesondere eine Silberbedampfung, durchgeführt. Die Metallbedampfung erfolgt gewöhnlich in einer Vakuumkammer, wobei die Metallatome bevorzugt in ionisierter Form auf die Gefäßprothese aufgetragen werden. Eine derartige Metallbedampfung kann beispielsweise mit Hilfe des IBAD-Verfahrens (ion-beam-assisted deposition-Verfahren) durchgeführt werden.

Die Gefäßprothese wird in einer weiteren Ausführungsform mit geeigneten Imprägniermaterialien beschichtet. Diesbezüglich wird vollständig auf die bisherige Beschreibung Bezug genommen.

In der Regel wird die Gefäßprothese nach ihrer Herstellung sterilisiert, vorzugsweise mit Ethylenoxid. Eine Ethylenoxid-Sterilisierung wird vorzugsweise dann durchgeführt, wenn es sich bei dem zumindest einen Verstärkungsmittel um einen Polypropylenfaden handelt.

Weitere Merkmale der Erfindung ergeben sich aus der nachfolgenden Beschreibung von bevorzugten Ausführungsformen in Form von Beispielen in Verbindung mit den Figuren und Unteransprüchen. Hierbei können die einzelnen Merkmale jeweils für sich alleine oder in Kombination miteinander verwirklicht sein. Die Figuren werden hiermit ausdrücklich durch Bezugnahme zum Inhalt der Beschreibung gemacht.

In den Figuren ist schematisch gezeigt:
- Figur 1:: die Beschichtung von Gefäßprothesen,
- Figur 2a,b:: die wendelförmige Verstärkung einer antimikrobiell beschichteten Gefäßprothese,
- Figur 3:: die wendelförmige Verstärkung einer unbeschichteten Gefäßprothese,
- Figur 4:: eine wendelförmig verstärkte, antimikrobiell beschichtete und imprägnierte Gefäßprothese,
- Figur 5:: eine ringförmig verstärkte, antimikrobiell beschichtete und imprägnierte Gefäßprothese.

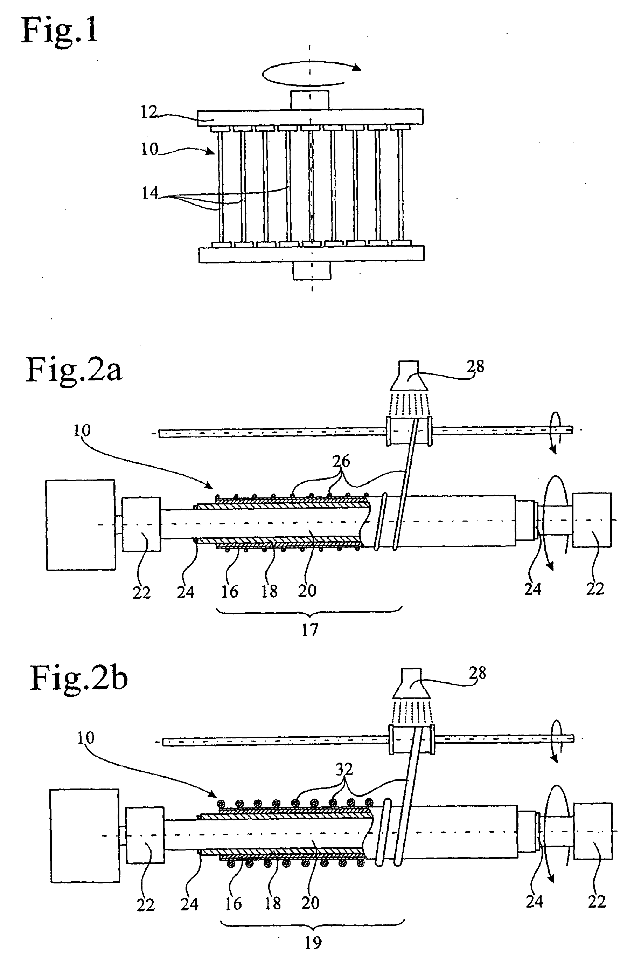

### Beispiel 1: Antimikrobielle Beschichtung von Gefäßprothesen (Figur 1)

Prothesen **10** aus Polyester (Polyethylenterephthalat) oder ePTFE werden in eine rotierbare Spannvorrichtung **12** eingespannt, so dass sie wie ein Bündel von parallelen Röhren **14** mit Zwischenabständen nebeneinander frei ragend hängen. Die Spannvorrichtung **12** wird in eine für die Durchführung des IBAD-Verfahrens geeignete Vakuumkammer eingesetzt, wobei eine Silberbedampfung der Gefäßprothesen **10** unter gleichzeitigem Beschuss mit Argonionen durchgeführt wird. Die Beschichtung wird solange durchgeführt, bis an der Außenseite der Gefäßprothesen 10 bzw. der dort liegenden Fasern eine Dicke der Silberschicht von ca. 130 nm erreicht ist. Wenn erwünscht, kann vorher eine Grundierung durch Bedampfung mit anderen Metallen, beispielsweise mit Titan und/oder Palladium, vorgenommen werden.

### Beispiel 2: Wendelförmige Verstärkung der antimikrobiell beschichteten Gefäßprothese (Figuren 2a und 2b)

Mit Silber beschichtete Gefäßprothesenrohlinge **16** aus Polyester (Polyethylenterephthalat) oder ePTFE werden auf ein Rohr **18** aus Polyethylen, beispielsweise mit einem Innen- und Außendurchmesser von 4,2 mm bzw. 6,0 mm, gezogen. Diese Einheit wird auf einen Stahlstab **20 ,** beispielsweise mit einem Außendurchmesser von 4 mm, aufgeschoben. Der Stahlstab **20** wird beidseitig mit Bohrfuttern **22** eingespannt und mit Kabelbindern **24** an beiden Enden fixiert. Ein silberfreier Schmelzfaden 26, beispielsweise ein mit einem Schmelzkleber ummantelter Polypropylen-Faden, wird mittels einer Hysterese-Bremse umgelenkt und gebremst (z.B. 5 Newton). Die Regeleinheit wird auf die nutzbare Länge, beispielsweise 1020 mm, eingestellt. Danach wird die Spindeleinstellung auf 90 Upm (Umdrehungen pro Minute) eingestellt. Die Heißlufteinheit **28** wird auf 180 °C eingestellt. Mit einer Zeitverzögerung von ca. 2 Minuten als Vorlaufzeit wird die Temperaturkonstanz gewährleistet. Die Laufzeit zum Aufbringen des Schmelzfadens **26** beträgt ca. 6 Minuten (Steigung: 4 mm). Nach einer Abkühlzeit von ca. 5 Minuten wird das Polyethylen-Rohr **18** (PE-Rohr) mit der wendelverstärkten Gefäßprothese **17** vom Stahlstab **20** abgezogen. Das Herunterziehen der Gefäßprothese **17** vom Polyethylen-Rohr **18** erfolgt durch Strecken, beispielsweise mit einer Greifzange.

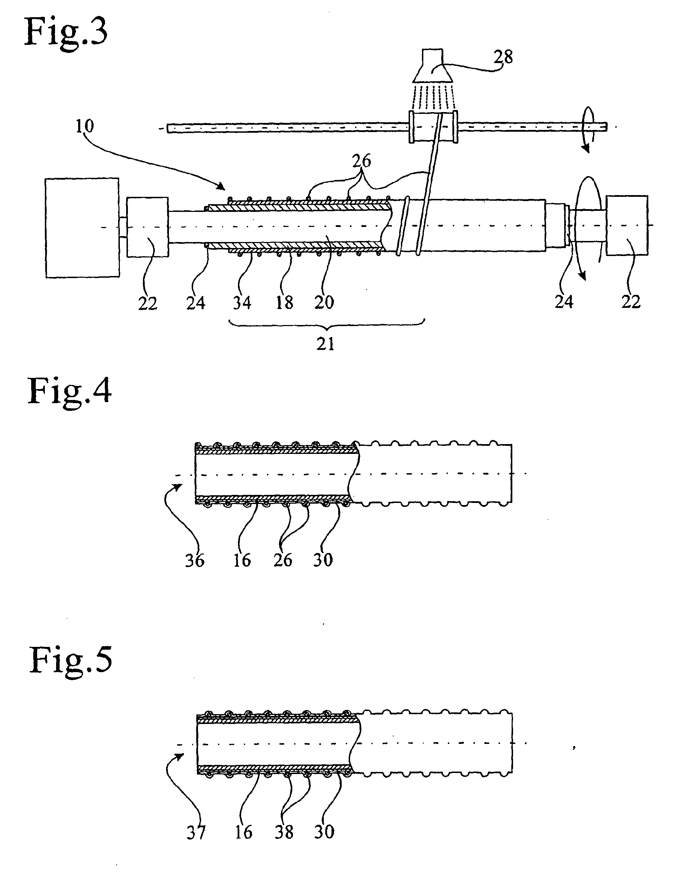

Anschließend wird die wendelförmig verstärkte beschichtete Gefäßprothese **17** einer erneuten Silberbeschichtung entsprechend Beispiel 1 unterworfen. Die so beschichtete Gefäßprothese wird aus der Spannvorrichtung entnommen und dann in üblicher Weise mit einem resorbierbaren Material **30** zumindest an ihrer Außenseite imprägniert (siehe Figur 4). Die Imprägnierung der Gefäßprothese **36** kann beispielsweise mit Gelatine vorgenommen werden, das zumindest teilweise mit Glutaraldehyd oder einem geeigneten Diisocyanat vernetzt wird. In die Beschichtungslösung können biologisch aktive Wirkstoffe eingebracht werden, um während der späteren Resorption der Schicht eine biologische Aktivität zu entwickeln.

Anstelle des in Beispiel 2 verwendeten silberfreien Schmelzfadens **26** kann grundsätzlich auch ein bereits mit Silber beschichteter Schmelzfaden **32** verwendet werden, wobei der Schmelzfaden **32** in diesem Fall beispielsweise ein Polypropylenfaden ist, der zuerst mit Silber und danach mit einem Schmelzkleber beschichtet wurde. Die so erhaltene wendelförmig verstärkte beschichtete Gefäßprothese **19** kann gegebenenfalls, beispielsweise wenn noch offen liegender Kleber ebenfalls mit einer antimikrobiellen Beschichtung versehen werden soll, einer nochmaligen Silberbedampfung unterworfen werden.

### Beispiel 3: Wendelförmige Verstärkung einer unbeschichteten Gefäßprothese (Figur 3)

Unbeschichtete Gefäßprothesenrohlinge **34** aus Polyester (Polyethylenterephthalat) oder ePTFE werden auf ein Rohr **18** aus Polyethylen, beispielsweise mit einem Innen- und Außendurchmesser von 4,2 mm bzw. 6,0 mm, gezogen. Diese Einheit wird auf einen Stahlstab **20,** beispielsweise mit einem Außendurchmesser von 4 mm, aufgeschoben. Der Stahlstab **20** wird beidseitig mit Bohrfuttern **22** eingespannt und mit Kabelbindern **24** an beiden Enden fixiert. Ein silberfreier Schmelzfaden **26** oder gegebenenfalls ein mit Silber beschichteter Schmelzfaden **32** (vgl. Figur 2b) wird mittels einer Hysterese-Bremse umgelenkt und gebremst (z.B. 5 Newton). Die Regeleinheit wird auf die nutzbare Länge, beispielsweise 1020 mm, eingestellt. Danach wird die Spindeleinstellung auf 90 Upm (Umdrehungen pro Minute) eingestellt. Die Heißlufteinheit **28** wird auf 180 °C eingestellt. Mit einer Zeitverzögerung von ca. 2 Minuten als Vorlaufzeit wird die Temperaturkonstanz gewährleistet. Die Laufzeit zum Aufbringen des Schmelzfadens **26** beträgt ca. 6 Minuten (Steigung: 4 mm). Nach einer Abkühlzeit von ca. 5 Minuten wird das Polyethylen-Rohr **18** (PE-Rohr) mit der wendelverstärkten unbeschichteten Gefäßprothese **21** vom Stahlstab **20** abgezogen. Das Herunterziehen der Gefäßprothese **21** vom Polyethylen-Rohr **18** erfolgt durch Strecken, beispielsweise mit einer Greifzange.

Anschließend wird die wendelförmig verstärkte Gefäßprothese **21** entsprechend Beispiel 1 antimikrobiell beschichtet. Die so beschichtete Gefäßprothese wird aus der Spannvorrichtung entnommen und, wie unter Beispiel 2 beschrieben, mit Gelatine imprägniert (Figur 4).

In Figur 5 ist schematisch eine ringförmig verstärkte, mit Silber beschichtete Gefäßprothese **37** dargestellt, die mit einem resorbierbaren Material **30,** beispielsweise vernetzter Gelatine, an ihrer Außenseite imprägniert ist. Die in Form von einzelnen Ringen vorliegenden Verstärkungsmittel **38** armieren die Gefäßprothese in regelmäßigen Abständen auf der Außenoberfläche der Prothesenwand.

### Beispiel 4: Produkteigenschaften einer Ausführungsform der erfindungsgemäßen Gefäßprothese

### Grundprothese:

- Polyester: gewirkt
- Bindung: Trikot-Trikot (gegenlegig)
- Garntiter: 50 dtex
- Filamentzahl 40
- Garnquerschnitt: rund
- Orientierungslinie (schwarz)
- Wasserdurchlässigkeit: 1500 ml/cm² * min

### Spirale:

- Material: monofiles Polypropylen, ummantelt mit einem Polybutylenreichen Schmelzkleber
- Durchmesser: 0,76 mm
- Beschichtungsgehalt: 64 %
- Lineare Reißkraft: 130 N
- Abstand der Spirale: 3,9 mm
- Haftfestigkeit der Spirale: 7,5 N

### Protheseneigenschaften:

- Wandstärke: 0,51 mm
- Radiale Reißkraft: 12,5 N

### Beschichtung/imprägnierung:

- Schichtdicke Silber: 125 nm
- Silbergehalt: 0,28 Gew.%
- Gelatinegehalt: 11,5 Gew.%
- Glyceringehalt: 12,7 Gew.%

## Patentansprüche

1. Gefäßprothese mit einer Prothesenwand, deren Außenoberfläche zumindest ein Verstärkungsmittel aufweist, wobei die Oberfläche der Prothesenwand und die Oberfläche des zumindest einen Verstärkungsmittels zumindest zum Teil eine antimikrobiell wirksame Schicht aufweisen, **dadurch gekennzeichnet, dass** das zumindest eine Verstärkungsmittel ein schmelzkleberummantelter Faden ist.

2. Gefäßprothese nach Anspruch 1, **dadurch gekennzeichnet, dass** das zumindest eine Verstärkungsmittel ein wendelförmig in Längsrichtung der Gefäßprothese verlaufendes Verstärkungsmittel ist.

3. Gefäßprothese nach Anspruch 1 oder 2, **dadurch gekennzeichnet, dass** die Oberfläche des zumindest einen Verstärkungsmittels vollständig beschichtet ist.

4. Gefäßprothese nach einem der vorhergehenden Ansprüche, **dadurch gekennzeichnet, dass** die Außenoberfläche der Prothese, insbesondere der Prothesenwand, vollständig beschichtet ist.

5. Gefäßprothese nach einem der vorhergehenden Ansprüche, **dadurch gekennzeichnet, dass** die antimikrobiell wirksame Schicht gleiche Schichtstärken auf der Außenoberfläche der Prothesenwand und der Oberfläche des zumindest einen Verstärkungsmittels aufweist.

6. Gefäßprothese nach einem der vorhergehenden Ansprüche, **dadurch gekennzeichnet, dass** die antimikrobiell wirksame Schicht eine Schichtdicke bis zu 400 nm aufweist.

7. Gefäßprothese nach einem der vorhergehenden Ansprüche, **dadurch gekennzeichnet, dass** das zumindest eine Verstärkungsmittel auf die Außenoberfläche der Prothesenwand aufgeklebt ist.

8. Gefäßprothese nach einem der vorhergehenden Ansprüche, **dadurch gekennzeichnet, dass** die Prothese eine textile Gefäßprothese, insbesondere eine gewirkte Gefäßprothese, ist.

9. Gefäßprothese nach einem der vorhergehenden Ansprüche **dadurch gekennzeichnet, dass** die antimikrobiell wirksame Schicht einen Anteil einer antimikrobiell wirksamen Substanz zwischen 0,05 und 1,0 Gew.%, insbesondere 0,10 und 0,40 Gew.%, aufweist, bezogen auf das Gesamtgewicht der Gefäßprothese.

10. Gefäßprothese nach einem der vorhergehenden Ansprüche, **dadurch gekennzeichnet, dass** die antimikrobiell wirksame Schicht eine metallische Schicht, insbesondere eine Schicht aus metallischem Silber, ist.

11. Verfahren zur Herstellung einer Gefäßprothese nach einem der vorhergehenden Ansprüche, umfassend die Schritte:
a) Aufbringen des zumindest einen Verstärkungsmittels auf die Außenoberfläche einer Gefäßprothese unter Ausbildung einer an ihrer Außenseite verstärkten Gefäßprothese,
b) Beschichten der Gefäßprothese mit einer antimikrobiell wirksamen Substanz vor und/oder nach dem Aufbringen des zumindest einen Verstärkungsmittels.

12. Verfahren zur Herstellung einer Gefäßprothese nach Anspruch 11 umfassend die Schritte:
a) Beschichten einer Gefäßprothese mit einer antimikrobiell wirksamen Substanz,
b) Aufbringen des zumindest einen Verstärkungsmittels auf die Außenoberfläche der beschichteten Gefäßprothese unter Ausbildung einer an ihrer Außenseite verstärkten beschichteten Gefäßprothese, wobei das zumindest eine Verstärkungsmittel vor dem Aufbringen bereits mit der antimikrobiell wirksamen Substanz beschichtet ist und/oder die beschichtete Gefäßprothese nach dem Aufbringen des zumindest einen Verstärkungsmittels nochmals beschichtet wird.

13. Verfahren nach Anspruch 11 oder 12, **dadurch gekennzeichnet, dass** das zumindest eine Verstärkungsmittel mittels eines Schmelzklebers auf die Außenoberfläche der Gefäßprothese aufgebracht wird.

14. Verfahren nach einem der Ansprüche 11 bis 13, **dadurch gekennzeichnet, dass** das Beschichten der Prothesenaußenoberfläche durch eine Silberbedampfung vorgenommen wird.

## Claims

1. Vascular prosthesis with a prosthesis wall whose outer surface has at least one reinforcing means, the surface of the prosthesis wall and the surface of the at least one reinforcing means being coated, at least in part, with an antimicrobial layer, **characterized in that** the at least one reinforcing means is a thread enveloped with a hot-melt adhesive.

2. Vascular prosthesis according to Claim 1, **characterized in that** the at least one reinforcing means is a reinforcing means that extends in a helical formation in the longitudinal direction of the vascular prosthesis.

3. Vascular prosthesis according to Claim 1 or 2, **characterized in that** the surface of the at least one reinforcing means is completely coated.

4. Vascular prosthesis according to one of the preceding claims, **characterized in that** the outer surface of the prosthesis, in particular of the prosthesis wall, is completely coated.

5. Vascular prosthesis according to one of the preceding claims, **characterized in that** the antimicrobial layer has identical layer thicknesses on the outer surface of the prosthesis wall and on the surface of the at least one reinforcing means.

6. Vascular prosthesis according to one of the preceding claims, **characterized in that** the antimicrobial layer has a layer thickness of up to 400 nm.

7. Vascular prosthesis according to one of the preceding claims, **characterized in that** the at least one reinforcing means is adhesively affixed to the outer surface of the prosthesis wall.

8. vascular prosthesis according to one of the preceding claims, **characterized in that** the prosthesis is a textile vascular prosthesis, in particular a knitted vascular prosthesis.

9. Vascular prosthesis according to one of the preceding claims, **characterized in that** the antimicrobial layer has a proportion of an antimicrobial substance of between 0.05 and 1.0% by weight, in particular of between 0.10 and 0.40% by weight, relative to the total weight of the vascular prosthesis.

10. Vascular prosthesis according to one of the preceding claims, **characterized in that** the antimicrobial layer is a metallic layer, in particular a layer of metallic silver.

11. Method for producing a vascular prosthesis according to one of the preceding claims, comprising the steps of:
a) applying the at least one reinforcing means to the outer surface of a vascular prosthesis so as to form a vascular prosthesis reinforced on its outside,
b) coating the vascular prosthesis with an antimicrobial substance before and/or after the application of the at least one reinforcing means.

12. Method for producing a vascular prosthesis according to Claim 11, comprising the steps of:
a) coating a vascular prosthesis with an antimicrobial substance,
b) applying the at least one reinforcing means to the outer surface of the coated vascular prosthesis to form a coated vascular prosthesis reinforced on its outside, the at least one reinforcing means already being coated with the antimicrobial substance before application and/or the coated vascular prosthesis being coated again after the application of the at least one reinforcing means.

13. Method according to Claim 11 or 12, **characterized in that** the at least one reinforcing means is applied to the outer surface of the vascular prosthesis by means of a hot-melt adhesive.

14. Method according to one of Claims 11 to 13, **characterized in that** the coating of the outer surface of the prosthesis is carried out by silver vapor deposition.

## Revendications

1. Prothèse vasculaire avec une paroi de prothèse, dont la surface extérieure présente au moins un moyen de renforcement, dans laquelle la surface de la paroi de prothèse et la surface dudit au moins un moyen de renforcement présentent au moins en partie une couche ayant un effet antimicrobien, **caractérisée en ce que** ledit au moins un moyen de renforcement est un fil enrobé de colle fusible.

2. Prothèse vasculaire selon la revendication 1, **caractérisée en ce que** ledit au moins un moyen de renforcement est un moyen de renforcement s'étendant en forme d'hélice dans la direction longitudinale de la prothèse vasculaire.

3. Prothèse vasculaire selon la revendication 1 ou 2, **caractérisée en ce que** la surface dudit au moins un moyen de renforcement est entièrement revêtue.

4. Prothèse vasculaire selon l'une quelconque des revendications précédentes, **caractérisée en ce que** la surface extérieure de la prothèse, en particulier de la paroi de prothèse, est entièrement revêtue.

5. Prothèse vasculaire selon l'une quelconque des revendications précédentes, **caractérisée en ce que** la couche ayant un effet antimicrobien présente les mêmes épaisseurs de couche sur la surface extérieure de la paroi de prothèse et sur la surface dudit au moins un moyen de renforcement.

6. Prothèse vasculaire selon l'une quelconque des revendications précédentes, **caractérisée en ce que** la couche ayant un effet antimicrobien présente une épaisseur de couche pouvant atteindre 400 nm.

7. Prothèse vasculaire selon l'une quelconque des revendications précédentes, **caractérisée en ce que** ledit au moins un moyen de renforcement est collé sur la surface extérieure de la paroi de prothèse.

8. Prothèse vasculaire selon l'une quelconque des revendications précédentes, **caractérisée en ce que** la prothèse est une prothèse vasculaire textile, en particulier une prothèse vasculaire tricotée.

9. Prothèse vasculaire selon l'une quelconque des revendications précédentes, **caractérisée en ce que** la couche ayant un effet antimicrobien présente une proportion d'une substance ayant un effet antimicrobien comprise entre 0,05 et 1,0 % en poids, en particulier entre 0,10 et 0,40 % en poids, rapportée au poids total de la prothèse vasculaire.

10. Prothèse vasculaire selon l'une quelconque des revendications précédentes, **caractérisée en ce que** la couche ayant un effet antimicrobien est une couche métallique, en particulier une couche en argent métallique.

11. Procédé de fabrication d'une prothèse vasculaire selon l'une quelconque des revendications précédentes, comprenant les étapes suivantes:
a) application dudit au moins un moyen de renforcement sur la surface extérieure d'une prothèse vasculaire avec formation d'une prothèse vasculaire renforcée sur son côté extérieur,
b) revêtement de la prothèse vasculaire avec une substance ayant un effet antimicrobien avant et/ou après l'application dudit au moins un moyen de renforcement.

12. Procédé de fabrication d'une prothèse vasculaire selon la revendication 11, comprenant les étapes suivantes:
a) revêtement d'une prothèse vasculaire avec une substance ayant un effet antimicrobien,
b) application dudit au moins un moyen de renforcement sur la surface extérieure de la prothèse vasculaire revêtue avec formation d'une prothèse vasculaire revêtue renforcée sur son côté extérieur, dans lequel ledit au moins un moyen de renforcement est déjà revêtu de la substance ayant un effet antimicrobien avant l'application et/ou la prothèse vasculaire revêtue est encore revêtue après l'application dudit au moins un moyen de renforcement.

13. Procédé selon la revendication 11 ou 12, **caractérisé en ce que** ledit au moins un moyen de renforcement est appliqué sur la surface extérieure de la prothèse vasculaire au moyen d'une colle fusible.

14. Procédé selon l'une quelconque des revendications 11 à 13, **caractérisé en ce que** le revêtement de la surface extérieure de la prothèse est effectué par une métallisation à la vapeur d'argent.
